# EUROPEAN PATENT SPECIFICATION

(11) **EP 4 192 324 B1**
(45) Date of publication and mention of the grant of the patent: **03.09.2025**
(21) Application number: 21791178.3
(22) Date of filing: 21.09.2021
(51) Int. Cl.: A61B 1/00, A61B 1/015, A61B 90/70, A61M 39/22

(54) **REUSE PREVENTION FOR SINGLE-USE VALVES FOR MEDICAL DEVICES**
WIEDERVERWENDUNGSVERHINDERUNG FÜR EINWEGVENTILE FÜR MEDIZINISCHE VORRICHTUNGEN
PRÉVENTION DE RÉUTILISATION POUR DES SOUPAPES À USAGE UNIQUE POUR DISPOSITIFS MÉDICAUX

(30) Priority: 29.09.2020 US 202063084863 P; 29.09.2020 US 202063084869 P
(43) Date of publication of application: 14.06.2023
(73) Proprietor: Boston Scientific Scimed, Inc., Maple Grove, MN 55311 (US)
(72) Inventor: LUIS, Brian, Maple Grove, Minnesota 55311 (US); LIMBERG, Pauline, R., Maple Grove, Minnesota 55311 (US); POLLOCK, Ryan, Vincent, William, Maple Grove, Minnesota 55311 (US); CUMMINGS, Nathan, T., Maple Grove, Minnesota 55311 (US)
(74) Representative: Vossius & Partner Patentanwälte Rechtsanwälte mbB
(86) International application number: PCT/US2021/051306
(87) International publication number: WO 2022/072179

(56) References cited:
- EP-B1- 3 073 889
- WO-A1-2020/243421
- US-B2- 6 874 517

## Description

### FIELD

The present disclosure relates generally to valves for medical devices. In particular, the present disclosure relates to cleaning valves for medical devices.

### BACKGROUND

Endoscopes include functionality to deliver fluids and suction at a site of a procedure. Tubing for delivering fluids and/or suction extends from a handle of the endoscope, through a shaft of the endoscope, and to a distal tip of the endoscope. During a procedure, body fluids, tissues, or other material can build up in the tubing. In order to aid in reprocessing of reusable endoscopes between procedures, pre-processing is performed in an endoscopy suite. For example, water or other fluids are flushed through the tubing after the endoscope is removed from a patient, in order to clear debris from the air/water and/or suction tubing. The cleaning valve may be inserted into an air/water valve cylinder (i.e., valve well) of an endoscope after the scope is removed from a patient and the procedure valve is removed from the valve cylinder. An operator may then depress a button of the cleaning valve for a predetermined amount of time to flush the air and/or water channels of the endoscope prior to further reprocessing of the endoscope. An important aspect of a single-use valve that it is not inadvertently reused. Another important aspect of a cleaning valve is that it is not confused with a procedural valve and inadvertently used in place thereof during a procedure when the scope is inserted within a patient. It is with all of the above considerations in mind that the improvements of the present disclosure may be useful.

EP 3 073 889 B1 relates to an air/water valve assembly for use in an endoscope or other medical equipment. The valve assembly includes a spool and a one-piece sealing member that includes a number of sealing rings. The spool can be injection molded, and the sealing member can be overmolded onto the spool. A spring, retainer, and housing are also included to form the valve assembly. As such, an air/water valve assembly may be made that can be used in a single medical procedure and then discarded in a cost-effective manner.

### SUMMARY

The invention is defined by the features of the independent claims. Preferred embodiments are defined in the dependent claims.

In one aspect, the present disclosure relates to a valve assembly for controlling fluid flow through valve wells. The valve assembly may include comprising an interface member and a valve stem to which the interface member is removably couplable. The valve stem may include a proximal portion, a distal portion, two or more orifices, and a lumen in fluid communication with first and second orifices of the two or more orifices. The valve assembly may be configured to transition from a first state to a second state in response to utilization of the valve assembly to control fluid flow through a valve well. The second state of the valve assembly may prevent utilization of the valve assembly to control fluid flow through valve wells.

In some embodiments, fluid communication between the first and second orifices via the lumen is blocked in the second state. In various embodiments, the lumen includes a transition deposit comprising a material configured to expand and fill a portion of the lumen when the material is exposed to a liquid. In many embodiments, the proximal and distal portions of the valve stem decouple to transition from the first state to the second state. In several embodiments, the proximal and distal portions of the valve stem are connected by a retention linkage and a separation linkage in the first state and the proximal and distal portions of the valve stem are connected by the retention linkage and not the separation linkage in the second state. In several such embodiments, the separation linkage or the retention linkage is disposed within the lumen. In some such embodiments, the separation linkage or the retention linkage is disposed outside of the lumen. In various such embodiments, the separation linkage comprises a loop disposed on a distal end of the proximal portion of the valve stem or on a proximal end of the distal portion of the valve stem. In multiple embodiments, utilization of the valve assembly to control fluid flow through a valve well comprises one or more of insertion of the valve stem into the valve well, controlling fluid flow through the valve well, and removal of the valve stem from the valve well. In some embodiments, the proximal portion of the valve stem is fixed relative to the distal portion of the valve stem by a molded weak point in the first state, and the proximal portion of the valve stem is slidably coupled to the distal portion of the valve stem in the second state. In some such embodiments, utilization of the valve assembly to control fluid flow through the valve well breaks the molded weak point to transition the valve assembly from the first state to the second state. In various embodiments, the proximal portion of the valve stem is fixed relative to the distal portion of the valve stem by a transition deposit in the first state, and the proximal portion of the valve stem is slidably coupled to the distal portion of the valve stem in the second state. In various such embodiments, exposure of the transition deposit to a liquid from utilization of the valve assembly to control fluid flow through the valve well dissolves the transition deposit to transition the valve assembly from the first state to the second state. Many embodiments include a plurality of radial legs with first and second ends and the first ends are pivotally connected to the valve stem. In many such embodiments, the second ends are retained proximate to the valve stem by a hat slidably coupled to the valve stem in the first state and the second ends are released by the hat in the second state.

In another aspect, the present disclosure relates to a system comprising a valve well and a valve assembly. The valve well may include a cavity with two or more ports. The valve assembly may be for controlling fluid flow through valve wells. The valve assembly may include a valve stem and an interface member. The valve stem may include a proximal portion, a distal portion, two or more orifices, and a lumen in fluid communication with first and second orifices of the two or more orifices. The interface member may be coupled to the valve well and the valve stem. The valve assembly may be configured to transition from a first state to a second state in response to utilization of the valve assembly to control fluid flow through the valve well. The second state of the valve assembly may prevent utilization of the valve assembly to control fluid flow through valve wells.

In some embodiments, utilization of the valve assembly to control fluid flow through the valve well comprises one or more of insertion of the valve stem into the valve well, controlling fluid flow through the valve well, and removal of the valve stem from the valve well. In various embodiments, fluid communication between the first and second orifices via the lumen is blocked in the second state. In many embodiments, the proximal and distal portions of the valve stem decouple to transition from the first state to the second state.

In yet another aspect, the present disclosure relates to a method. The method may include one or more of: exposing a valve assembly to a valve well; and transitioning the valve assembly from a first state to a second state by exposing the valve assembly to the valve well, wherein the second state of the valve assembly prevents utilization of the valve assembly to control fluid flow through valve wells. In some embodiments, exposing the valve stem to the valve well comprises one or more of inserting the valve stem into the valve well, controlling flow of a fluid through the valve well with the valve stem, and removing the valve stem from the valve well:

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. In the figures:
**FIG. 1** illustrates an exemplary valve assembly in conjunction with a valve well according to one or more embodiments disclosed hereby.
**FIGS. 2A** and **2B** illustrate various aspects of an exemplary valve assembly in conjunction with a valve well according to one or more embodiments disclosed hereby.
**FIG. 3A** illustrates various aspects of an exemplary valve assembly in conjunction with a valve well according to one or more embodiments disclosed hereby.
**FIG. 3B** illustrates various aspects of an exemplary valve assembly in conjunction with a valve well according to one or more embodiments disclosed hereby.
**FIGS. 4A-4C** illustrate various aspects of exemplary valve stem portions according to one or more embodiments disclosed hereby.
**FIGS. 5A-5C** illustrate various aspects of exemplary valve stem portions according to one or more embodiments disclosed hereby.
**FIGS. 6A** and **6B** illustrate an exemplary valve stem with radial legs according to one or more embodiments disclosed hereby.

### DETAILED DESCRIPTION

A medical cleaning valve (or cleaning valve) may be configured to provide cleaning functionality to air and water channels of an endoscope. In a first configuration, the cleaning valve may provide a continuous feed of air to both air and water channels in a handle and shaft of an endoscope, and through an air/water nozzle at the distal end of the endoscope. In a second configuration, the cleaning valve may feed water into the air channel in the handle and shaft of the endoscope, and through the air nozzle at the distal end of the endoscope. The cleaning valves (or valves) of the current disclosure are generally single-use devices (SUDs) and therefore disposable. Reusing SUDs that are not designed for reprocessing and reuse can result in unnecessary exposure to bacteria. Accordingly, many valves (or valve assemblies) disclosed hereby may be configured to transition from a first state to a second state in response to utilization of the valve to control fluid flow through a valve well. The transition from the first state to the second state may prevent reuse in of one or more valves disclosed hereby. For instance, a lumen between first and second orifices in a valve stem may be blocked or disconnected.

Further, the valve may be made from a limited number of parts and materials, e.g., to limit its cost, so that it may be economically disposable. For example, an interface member may seal an opening to the lumen of the valve. In yet another example, the valve may have a single elastomeric component, or spring cap. Additionally, cleaning valves may have a similar appearance to procedural valves. However, using a cleaning valve in place of a procedural valve may result in fluid flow through an incorrect endoscope channel, e.g., liquid being delivered through the air channel. Also, using a cleaning valve in place of a procedural valve may result in continuous insufflation of air into a patient, such as via both the air and water channels. Accordingly, one or more embodiments disclosed hereby may include cleaning valves with features and/or components that facilitate differentiating them from procedural valves.

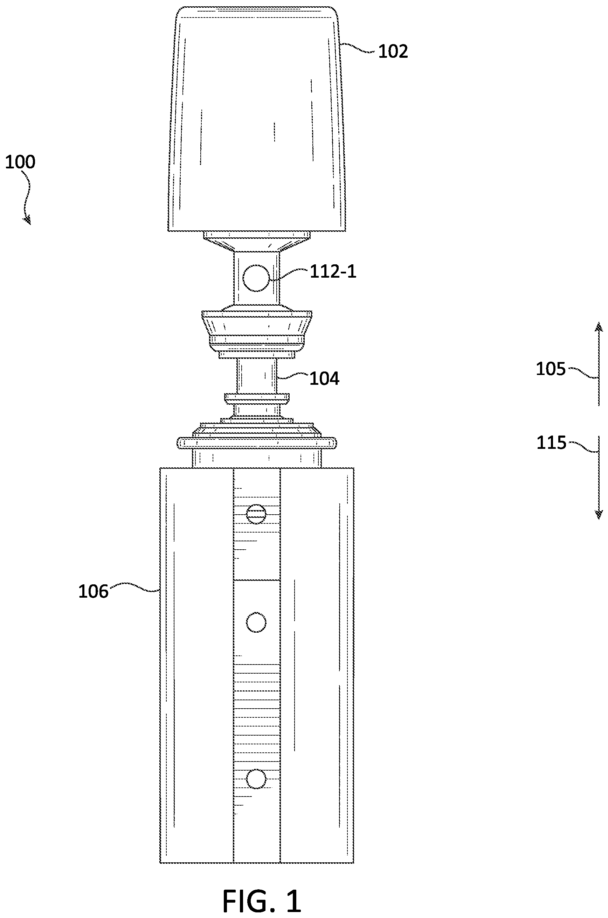

It may be understood that the disclosure included herein is exemplary and explanatory only and is not restrictive. As used herein, the terms "comprises," "comprising," or any other variation thereof, are intended to cover a non-exclusive inclusion, such that a process, method, article, or apparatus that comprises a list of elements does not include only those elements, but may include other elements not expressly listed or inherent to such process, method, article, or apparatus. The term "exemplary" is used in the sense of "example," rather than "ideal." As used herein, the term "proximal" means a direction closer to a surface used by an operator for operating a valve (e.g., an interface member, a user interface, a button) and the term "distal" means a direction away from the surface used by an operator for operating a valve (e.g., a button). Regarding FIGS. 1-6B, "proximal" may refer to towards the top of the drawing sheet and "distal" may refer to towards the bottom of the drawing sheet. Although endoscopes are referenced herein, reference to endoscopes or endoscopy should not be construed as limiting the possible applications of the disclosed aspects. For example, the disclosed aspects may be used with duodenoscopes, bronchoscopes, ureteroscopes, colonoscopes, catheters, diagnostic or therapeutic tools or devices, or other types of medical devices. Additionally, although cleaning valves are referenced herein, reference to cleaning valves should not be construed as limiting the possible applications of the disclosed aspects. For example, the disclosed aspects may be used with a variety of medical valves for controlling the flow of fluids.

Reference is now made to the drawings, wherein like reference numerals are used to refer to like elements throughout. In the following description, for purpose of explanation, numerous specific details are set forth in order to provide a thorough understanding thereof. It may be evident, however, that the novel embodiments can be practiced without these specific details. In other instances, well known structures and devices are shown in block diagram form to facilitate a description thereof. The intention is to cover all modification, equivalents, and alternatives within the scope of the claims.

**FIG. 1** illustrates various aspects of a valve assembly 100 in conjunction with a valve well 106 according to one or more embodiments of the present disclosure. The valve assembly 100 may include an interface member 102 and a valve stem 104 with orifice 112-1. More generally, a valve assembly may refer to an interface member in conjunction with a valve stem. In various embodiments, the valve assembly 100 may be inserted into and/or coupled with the valve well 106. The valve assembly 100 and valve well 106 may be oriented with proximal end 105 and distal end 115. In many embodiments, the interface member 102 may be interacted with to move the valve stem 104 proximally or distally within the valve well 106 to control the flow of fluid through the valve well 106. For instance, valve assembly 100 may be utilized to route water to flush out one or more tubes and/or lumens in an endoscopic system. In some embodiments, FIG. 1 may include one or more components that are the same or similar to one or more other components of the present disclosure. Further, one or more components of FIG. 1, or aspects thereof, may be incorporated into other embodiments of the present disclosure without departing from the scope of this disclosure. Embodiments are not limited in this context.

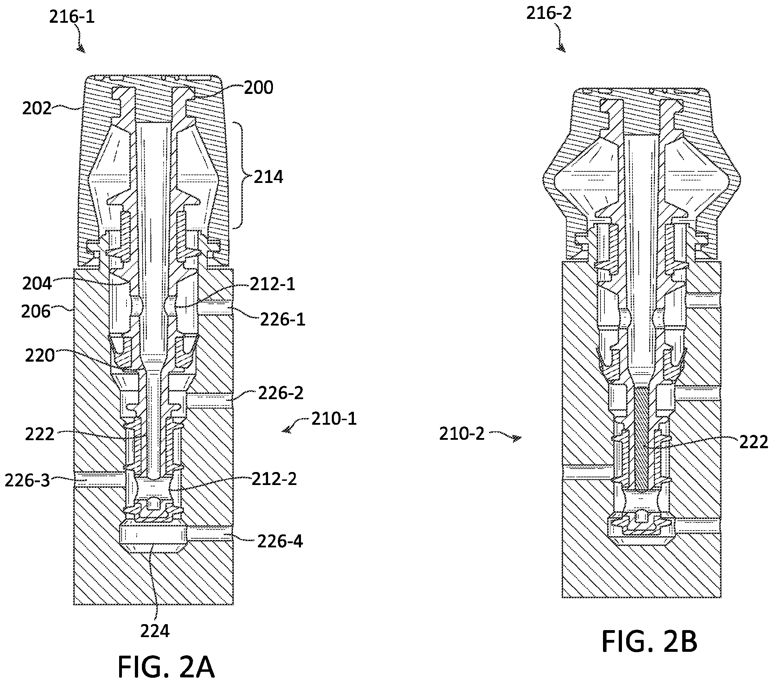

**FIGS. 2A** and **2B** illustrate various aspects of a valve assembly 200 in conjunction with a valve well 206 according to one or more embodiments of the present disclosure. More specifically, FIG. 2A includes a cross-sectional view of valve assembly 200 in a first configuration 216-1 and FIG. 2B includes a cross-sectional view of valve assembly 200 in a second configuration 216-2. The valve assembly 200 may include an interface member 200 and a valve stem 204. The interface member 200 may include an elastic section 214. The valve stem 204 may include a first orifice 212-1 and a second orifice 212-2 connected by a lumen 220. The valve well 206 may include a cavity 224 with ports 226-1, 226-2, 226-3, 226-4. In some embodiments, FIGS. 2A and/or 2B may include one or more components that are the same or similar to one or more other components of the present disclosure. For example, valve stem 204 may be the same or similar to valve stem 104. Further, one or more components of FIGS. 2A and/or 2B, or aspects thereof, may be incorporated into other embodiments of the present disclosure without departing from the scope of this disclosure. For example, the transition deposit 222 may be incorporated into valve assembly 100 without departing from the scope of this disclosure. Embodiments are not limited in this context.

In the first configuration 216-1, the valve assembly 200 may be coupled to the valve well 206 with no user input. In the second configuration 216-2, the valve assembly 200 may be coupled to the valve well 206 with user input that compresses an elastic section 214 of interface member 202 and causes the valve stem 204 to move distally within the valve well 206. In many embodiments, the second configuration may cause water input via port 226-3 to feed water, via port 226-1, into the air channel in a handle and shaft of an endoscope (not shown), and through the air nozzle at the distal end of the endoscope.

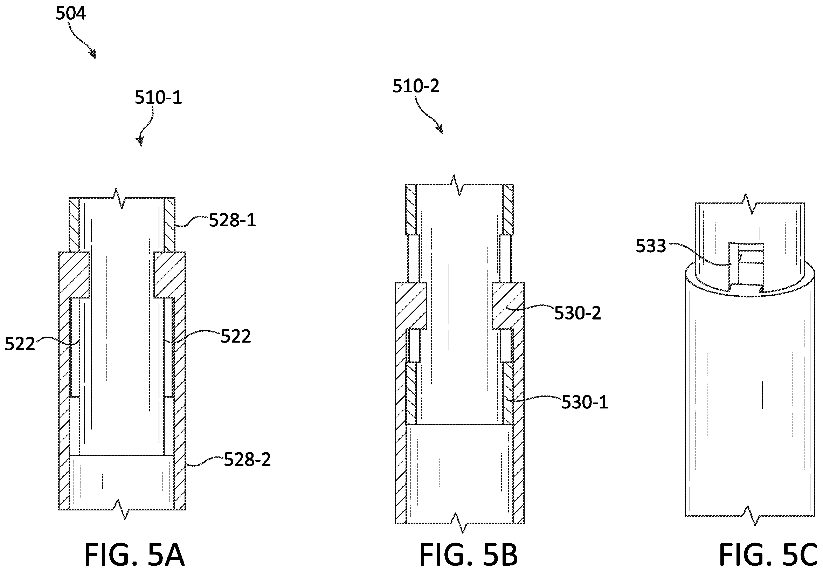

Additionally, FIGS. 2A and 2B illustrate the valve assembly 200 in different states. FIG. 2A illustrates the valve assembly 200 in a first state 210-1 and FIG. 2B illustrates the valve assembly 200 in a second state 210-2. In the first state 210-1, transition deposit 222 may be disposed in the lumen 220 in an unexposed state and in the second state 210-2 may transition to an exposed state to block the flow of water through the lumen 220. For example, transition deposit 222 may expand due to exposure to a fluid. Accordingly, the transition material may include a material that expands and hardens after exposure to a certain amount of volumetric fluid flow, such as Porex^{®}. In other embodiments, the transition deposit may contract and/or dissolve due to exposure to a fluid (see e.g., FIGS. 5A-5C). In various embodiments, the transition deposit 222 may comprise a cylindrical insert in the lumen 220. In some embodiments, the transition deposit 222 may be a coating, such as by being powder coated onto a surface.

More generally, one or more components of valve assemblies disclosed hereby may be configured to transition from the first state to the second state in response to utilization of the valve assembly to control fluid flow through a valve well. The transition from the first state to the second state may prevent reuse in of one or more valve assemblies disclosed hereby. For example, one or more components of the valve assembly may be altered, such as by lengthening, transitioning from a first state to a second state (e.g., chemically), shortening, widening, detaching, and the like.

In several embodiments the amount of volumetric flow required for the transition deposit 222 to block the lumen 220 may be correlated to the particular use and/or amount of time a valve assembly 220 is used. For example, properly cleaning (i.e., reprocessing) an endoscope may require water flushing through the air channel of the endoscope for at least 30 seconds, requiring the valve assembly 200 to remain in the second configuration 216-2 for at least 30 seconds. Accordingly, the transition deposit 222 may be configured to require an amount of volumetric flow to transition to the second state 210-2 that is equivalent to 30 seconds of flow through the air channel. In many embodiments, this technique may simplify or improve cleaning, by requiring the valve assembly 200 just be depressed until it is fully transitioned to the second state 210-2 instead of requiring 30 seconds be timed.

In another example, a properly cleaning an endoscope may require a specific volume of water be flushed through the air channel of the endoscope. In such other examples, the transition deposit 222 may be configured to block the lumen 220 after the specific volume of water is flushed through the air channel. In this example, cleaning efficiency/reliability may be improved due to the state transition occurring directly due to volumetric flow, as opposed to an amount of time. This may be due to one or more factors, such as varying flow rates between different endoscopes or varying pressures or flow rates provided by different processor and/or insufflator setups. The processor and/or insufflator setups may comprise the source of flow fed to the valve well of the endoscope. It will be appreciated that although FIG. 2B illustrates state 210-2 while the valve assembly 200 is in the second configuration 216-2, the transition to the second state 210-2 may occur, at least partially, after the valve assembly 200 returns to the first configuration 216-1 (e.g., due to elastic section 214 comprising a biasing member to return the valve assembly 200 to the first configuration 216-1 in the absence of external input).

Utilization of a valve assembly (e.g., valve assembly 200) to control fluid flow through a valve well may include one or more of coupling the interface member to the valve well, transitioning the interface member from a first configuration to a second configuration, insertion of the distal end of the valve assembly into the proximal end of the valve well, controlling the flow of fluid through the valve well with the valve assembly 100, and removing the valve assembly from the valve well. In many embodiments, the utilization of the valve assembly to control fluid flow through a valve well may cause the transition from the first state to the second state. For example, exposing transition deposit 222 to water while using the valve assembly 200 to control fluid flow through the valve well 206 (e.g., to flush an air channel) may cause transition deposit 222 to transition from the first state 210-1 to the second state 210-2.

In various embodiments, the elastic section 214 may function as a biasing member. For example, elastic section 214 may bias the interface member 202 (and the corresponding valve assembly) into the first configuration 216-1. In other embodiments, the elastic section 214 may not include a biasing member. In such other embodiments, a spring may be used as the biasing member. In some embodiments, the walls of the elastic section 214 may have a varying in thickness and or other structures to bias interface member 202 into the first configuration 216-1. In some embodiments, the first configuration 216-1 may comprise a standby configuration and the second configuration 216-2 may comprise an actuated configuration.

In several embodiments, the interface member 202 may couple with the valve well. In several such embodiments, the interface member 202 may exert a force against the valve well 206 to bias the interface member in the first configuration 216-1. In many embodiments, the second configuration 216-2 may cause fluid to flow through the valve well 206. On the other hand, the interface member 202 (and remainder of valve assembly) may be coupled to the valve well 206 in the first configuration 216-1 without causing fluid to flow through the valve well 206. In many embodiments, the interface member 202 may be displaced distally to transition from the first configuration 216-1 to the second configuration 216-2. Further, the distal displacement of the interface member 202 moves the valve stem 204 distally within the valve well 206. In some embodiments, interface members may include one or more parts. For example, a finger of an operator may interact with a first part of the interface member and a second part of the interface member may couple with the valve well.

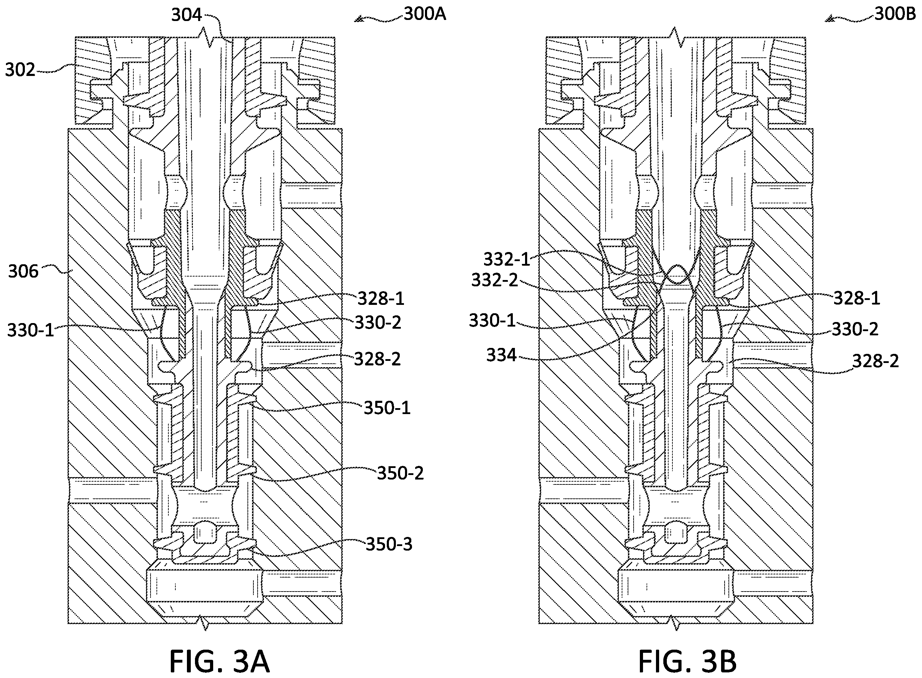

**FIGS. 3A** and **3B** illustrate various aspects of a valve assemblies 300A, 300B in conjunction with a valve well 306 according to one or more embodiments of the present disclosure. FIG. 3A includes valve assembly 300A and valve well 306. Valve assembly 300A may include interface member 302 and valve stem 304. Valve stem 304 may include first stem portion 328-1 and second stem portion 328-2 connected by retention linkages 330-1, 330-2. The Valve assembly 300B may be the same or similar to valve assembly 300A. In FIG. 3B, valve assembly 300B may include contact surface 334 and separation linkages 332-1, 332-2 in addition to the components of valve assembly 300A. Although not labeled, in some embodiments, valve assembly 300A may include contact surface 334. In various embodiments, the first and second stem portions 328-1, 328-2 may be able to move relative to each other, such as by telescoping, to transition from a first state to a second state. In some embodiments, FIGS. 3A and/or 3B may include one or more components that are the same or similar to one or more other components of the present disclosure. For example, valve stem 304 may be the same or similar to valve stem 204. Further, one or more components of FIGS. 3A and/or 3B, or aspects thereof, may be incorporated into other embodiments of the present disclosure without departing from the scope of this disclosure. For example, the retention linkages 330-1, 330-2 may be incorporated into valve assembly 100 without departing from the scope of this disclosure. Embodiments are not limited in this context.

In many embodiments, the first and second stem portions 328-1, 328-2 may be able to move relative to each other, such as by telescoping, to transition from a first state to a second state. For instance, one or more weak points (e.g., a thin wall section, perforations, a transition deposit, poor filling, or weakened post molding) may be included, such as via molding, additive manufacturing, subtracting manufacturing, and/or powder coating, at the junction between the first and second stem portion 328. In some such instances, the weak points may allow the stem portions 328 to move relative to one another, such as during removal from the valve well or returning from an actuated configuration (e.g., the second configuration) to a standby configuration (e.g., the first configuration). In several embodiments, the retention linkages 330-1, 330-2 may keep the first and second stem portions 328 connected in the second state. In several such embodiments, the retention linkages 330 may prevent stem portion 328-2 from remaining in the valve well 306 when the valve assembly is removed. Frictional forces corresponding to radial seals 350-1, 350-2, 350-3 on the valve stem 304 contacting the cavity of the valve well may cause the first and second stem portions 328 to separate during removal from the valve well.

In one or more embodiments disclosed hereby, the first and second stem portions 328 may have loops (e.g., separation linkages) that interlock to fit each other. When pressed together (e.g., in the actuated configuration), the loops may press into solid surfaces of the opposing component (e.g., contact surface 334, contact surface 434). When the proximal portion is pulled away from the distal portion during removal, a perforated break point included on one or more of the loops may allow them to come apart. In order to retain the distal stem portion during removal and prevent it from being left in the valve well, the proximal and distal stem portions may be connected by retention linkages.

The separation linkages 332-1, 332-2 of FIG. 3B may comprise loops that break and/or become disconnected in the second state. For example, one or more of the separation linkages 332 may break apart when the valve assembly 300B is removed from the valve well 306. In various embodiments, the separation linkage 332-1 of stem portion 328-1 may push against the contact surface 334 of stem portion 328-2 when the interface member is depressed to transition the valve assembly 300B from the standby configuration to the actuated configuration. In the illustrated embodiment, the separation linkages 332 may be disposed inside of the lumen of the valve stem and the retention linkages 330 may be disposed on the exterior of the valve stem. However, valve assemblies may include one or more retention linkages and/or one or more separation linkages connecting stem portions 328 via a variety of locations without departing from the scope of this disclosure. In various embodiments, the linkages may include or be referred to as leashes. In some embodiments, the linkages may comprise a polymer and/or a metal (e.g., nitinol). In several embodiments, the linkages may be integrally molded with the valve stem.

In many embodiments, one or more components and/or features disclosed hereby may be used to differentiate cleaning valves from procedural valves. For example, retention linkages 330-1, 330-2 may differentiate valve assembly 300 from procedural valve assemblies. In another example, the interface member 302 may include one or more features for distinction, such as raised surfaces, colors, warning labels, and the like.

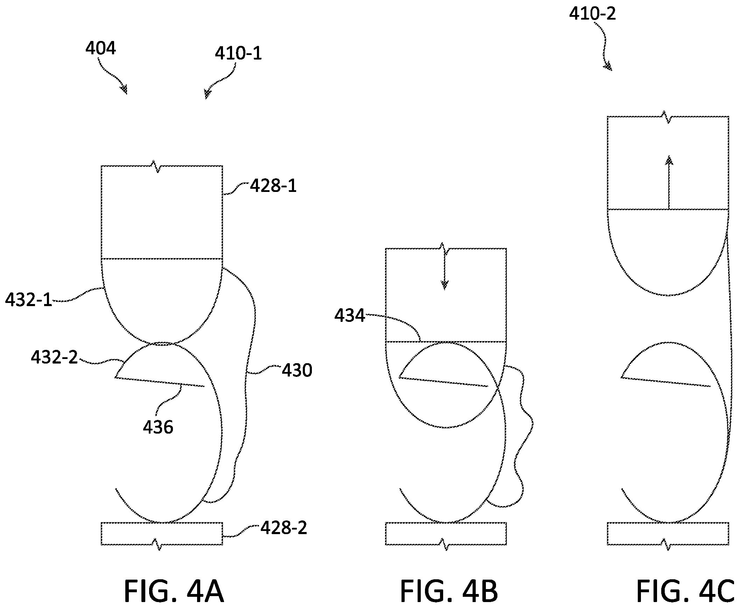

**FIGS. 4A-4C** illustrate various aspects of valve stem portions 428-1, 428-2 of valve stem 404 according to one or more embodiments of the present disclosure. FIGS. 4A-4C illustrate an alternative embodiment of a valve stem with first and second stem portions 428-1, 428-2, retention linkage 430, and separations linkages 432-1, 432-2. In the illustrated embodiment, separation linkage 432-2 is attached to the proximal end of stem portion 428-2 and includes arm 436. In some embodiments, arm 436 may pivot and/or be biased into a certain position by a biasing member (e.g., spring). Separation linkage 432-1 is attached to the distal end of stem portion 428-1, which includes contact surface 434. In some embodiments, FIGS. 4A-4C may include one or more components that are the same or similar to one or more other components of the present disclosure. For example, stem portions 428-1, 428-2 may be the same or similar to stem portions 328-1, 328-2. Further, one or more components of FIGS. 4A-4C, or aspects thereof, may be incorporated into other embodiments of the present disclosure without departing from the scope of this disclosure. For example, the separation linkages 432-1, 432-2 may be incorporated into valve stem 304 without departing from the scope of this disclosure. Embodiments are not limited in this context.

In various embodiments, FIGS. 4A-4C illustrates the first stem portion 428-1 with a strong, non-perforated loop and the second stem portion 428-2 with a spring-loaded gate (i.e., arm 436) loop (e.g., separation linkage 432-2), like a carabiner except the arm 436 may be biased open instead of in the closed position. More specifically, FIG. 4A illustrates the valve stem 404 in the first state 410-1, which may correspond to the standby configuration; FIG. 4B illustrates the valve stem 404 in a transition state, which may correspond to the actuated configuration; and FIG. 4C illustrates the valve stem 404 in the second state 410-2, which may occur when the valve stem 404 is removed from a valve well after the transition state. In the transition state, contact surface 434 may push against a proximal end of separation linkage 432-2 to push the second stem portion 428-2 distally in a valve well. Further, the distal end of separation linkage -432-1 may push past (e.g., by pushing open) the arm 436 and allow the arm 436 to reclose. When the valve stem is removed the reclosed arm 436 forces the separation linkages 432 to separate.

**FIGS. 5A-5C** illustrate various aspects of valve stem portions 528-1, 528-2 according to one or more embodiments of the present disclosure. The valve stem 504 may include stem portions 528-1, 528-2 and retention linkages 530-1, 530-2. FIG. 5A illustrates valve stem 504 in a first state 510-1 and including transition deposit 522. FIGS. 5B and 5C illustrate valve stem 504 in a second state 510-2. In some embodiments, FIGS. 5A-5C may include one or more components that are the same or similar to one or more other components of the present disclosure. For example, stem portions 528-1, 528-2 may be the same or similar to stem portions 328-1, 328-2. Further, one or more components of FIGS. 5A-5C, or aspects thereof, may be incorporated into other embodiments of the present disclosure without departing from the scope of this disclosure. For example, the stem portions 328-1, 328-2 may be incorporated into valve stem 104 without departing from the scope of this disclosure. Embodiments are not limited in this context.

In the first state 510-1, transition deposit 522 may lock the stem portions 528-1, 528-2 in a compressed position relative to each other. However, in the second state 510-2, transition deposit 522 may be absent and the stem portions 528 may be in an extended position relative to each other. Accordingly, in the absence of transition deposit 522, stem portions 528 may move relative to one another, such as by telescoping. In many embodiments, transition deposit 522 may operate in an opposite manner as the transition deposit 222, such as by dissolving when exposed to a fluid.

In many embodiments, the extended position may expose one or more radial openings 533 to prevent reuse of the valve stem 504. In some embodiments, the valve stem 504 may include one or more features to lock the stem portions 528 in the extended state. For example, a transition deposit similar to transition deposit 222 may be used to lock the stem portions 528 in the extended state. In other embodiments, the stem portions 528 may freely move relative to one another in the second state 510-2. In various embodiments, controlling fluid flow through a valve well may cause transition deposit 522 to dissolve (or at least weaken), allowing the valve stem to transition into state 510-2 when it is removed from a valve well. In several embodiments, transition deposit 522 may comprise a glue, such as polyvinyl acetate.

Retention linkages 530-1, 530-2 may include corresponding features that prevent stem portions 528 from separating when they are removed from a valve well. For example, retention linkages 530 may include a collar and/or ledge. In another example, retention linkage 530-1 may include a channel and retention linkage 530-2 may include a protrusion that moves within the channel.

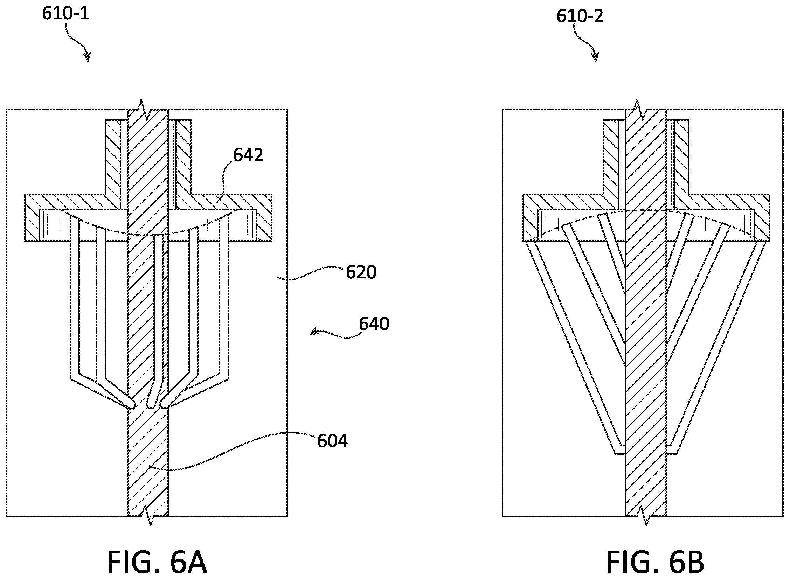

**FIGS. 6A** and 6**B** illustrate a valve stem 604 with radial legs 640 according to one or more embodiments of the present disclosure. The valve stem 604 may additionally include hat 642. FIG. 6A may illustrate the valve stem 604 in a first state 610-1 with the hat 642 retaining the one or more radial legs 640 proximate the valve stem 604. FIG. 6B may illustrate the valve stem 604 in a second state 610-2 with the radial legs 640 released by the hat 642. In some embodiments, FIGS. 6A and/or 6B may include one or more components that are the same or similar to one or more other components of the present disclosure. For example, valve stem 604 may be the same or similar to valve stem 104. Further, one or more components of FIGS. 6A and/or 6B, or aspects thereof, may be incorporated into other embodiments of the present disclosure without departing from the scope of this disclosure. For example, the radial legs 640 may be incorporated into valve assembly 200 without departing from the scope of this disclosure. Embodiments are not limited in this context.

In various embodiments, valve stem 604 may include one or more radial legs 640 with a first end connected to the valve stem 604. The hat 642 may retain the second end of the radial legs 640 proximate the shaft of the valve stem 604 until the valve stem is depressed. In various embodiments, the hat 642 may include one or more of a cylindrical cuff, a containing tray/box/guard, and the like. In many embodiments, the radial legs 640 are in a shortened configuration when they are retained by hat 642. For example, the radial legs may include a bend or a bow in state 610-1. In some such examples, the bend or the bow may cause the radial legs 640 to expand radially and extend proximally when released by the hat. In some embodiments, biasing members, such as springs, may be used to cause the radial legs 640 to expand radially and extend proximally.

In the second state 610-2, the legs may extend and lock, such as against the hat 642, to cause the valve stem 604 to lengthen and become inoperable to control the flow of fluid through a valve well. For example, the radial legs 640 may be fixed to the valve stem 604 and hat 642 may be slidably coupled to the valve stem 604. Accordingly, as the valve stem 604 is depressed distally (e.g., to transition into the actuated configuration) the second ends of the radial legs 640 may move distally enough to no longer be retained by the hat 640. When the radial legs 640 are not retained by the hat 642, the radial legs 640 may lengthen. When the valve stem 604 returns proximally (e.g., to transition back the standby configuration) the radial legs 640 may lock, in the lengthened position, against the hat 642 to increase the overall length of the valve stem 604.

This application relates to U.S. Patent Application No. 16/868,325, titled "Devices, Systems, Methods, and Designs for Medical Cleaning Valves, filed May 6, 2020.

This application relates to U.S. Patent Application No. 16/868,329, titled "Devices, Systems, and Methods for Medical Cleaning Valves, filed May 6, 2020.

## Claims

1. A valve assembly (100, 200, 300A, 300B) for controlling fluid flow through valve wells, the valve assembly comprising:
an interface member (102, 202, 302); and
a valve stem (104, 204, 304, 404, 504, 604) to which the interface member is removably couplable, the valve stem including a proximal portion (328-1, 428-1, 528-1), a distal portion (328-2, 428-2, 528-2), two or more orifices (112, 212), and a lumen (220) in fluid communication with first and second orifices of the two or more orifices,
wherein the valve assembly is configured to transition from a first state (210-1, 410-1, 510-1, 610-1) to a second state (210-2, 410-2, 510-2, 610-2) in response to utilization of the valve assembly to control fluid flow through a valve well (106, 206, 306), and wherein the second state of the valve assembly prevents re-utilization of the valve assembly to control fluid flow through valve wells,
wherein fluid communication between the first and second orifices via the lumen is blocked in the second state.

2. The valve assembly of claim 1, wherein the lumen (220) includes a transition deposit (222, 522), comprising a material configured to expand and fill a portion of the lumen when the material is exposed to a liquid.

3. The valve assembly of any of claims 1 or 2, wherein the proximal and distal portions (328-1, 428-1, 528-1, 328-2, 428-2, 528-2) of the valve stem decouple to transition from the first state (410-1, 510-1) to the second state (410-2, 510-2).

4. The valve assembly of any of claims 1 to 3, wherein the proximal and distal portions (328-1, 428-1, 328-2, 428-2) of the valve stem are connected by a retention linkage (330-1, 330-2, 430) and a separation linkage (332-1, 332-2, 432-1, 432-2) in the first state (410-1) and the proximal and distal portions of the valve stem are connected by the retention linkage and not the separation linkage in the second state (410-2).

5. The valve assembly of claim 4, wherein the separation linkage (332-1, 332-2, 432-1, 432-2) or the retention linkage (330-1, 330-2, 430) is disposed within the lumen (220).

6. The valve assembly of any of claims 4 to 5, wherein the separation linkage or the retention linkage s disposed outside of the lumen (220).

7. The valve assembly of any of claims 4 to 6, wherein the separation linkage (332-1, 332-2, 432-1, 432-2) comprises a loop disposed on a distal end of the proximal portion (328-1, 428-1) of the valve stem or on a proximal end of the distal portion (328-2, 428-2) of the valve stem.

8. The valve assembly of any of claims 1 to 7, wherein utilization of the valve assembly to control fluid flow through a valve well (106, 206, 306) comprises one or more of insertion of the valve stem (104, 204, 304, 404, 504, 604) into the valve well, controlling fluid flow through the valve well, and removal of the valve stem from the valve well.

9. The valve assembly of any of claims 1 to 8, wherein the proximal portion (328-1) of the valve stem is fixed relative to the distal portion (328-2) of the valve stem by a molded weak point in the first state, and the proximal portion of the valve stem is slidably coupled to the distal portion of the valve stem in the second state.

10. The valve assembly of claim 9, wherein utilization of the valve assembly to control fluid flow through the valve well (306) breaks the molded weak point to transition the valve assembly from the first state to the second state.

11. The valve assembly of any of claims 1 to 10, wherein the proximal portion (528-1) of the valve stem is fixed relative to the distal portion (528-2) of the valve stem by a transition deposit (522) in the first state (510-1), and the proximal portion of the valve stem is slidably coupled to the distal portion of the valve stem in the second state (510-2).

12. The valve assembly of claim 11, wherein exposure of the transition deposit (522) to a liquid from utilization of the valve assembly to control fluid flow through the valve well dissolves the transition deposit to transition the valve assembly from the first state (510-1) to the second state (510-2).

13. The valve assembly of any of claims 1 to 12, comprising a plurality of radial legs (640) with first and second ends, wherein the first ends are pivotally connected to the valve stem (604).

14. The valve assembly of claim 13, where the second ends are retained proximate to the valve stem (604) by a hat (642) slidably coupled to the valve stem in the first state (610-1) and the second ends are released by the hat in the second state (610-2).

## Patentansprüche

1. Ventilanordnung (100, 200, 300A, 300B) zum Steuern eines Fluidstroms durch Ventilschächte, wobei die Ventilanordnung aufweist:
ein Schnittstellenelement (102, 202, 302); und
einen Ventilschaft (104, 204, 304, 404, 504, 604), mit dem das Schnittstellenelement lösbar gekoppelt werden kann, wobei der Ventilschaft einen proximalen Abschnitt (328-1, 428-1, 528-1), einen distalen Abschnitt (328-2, 428-2, 528-2), zwei oder mehr Öffnungen (112, 212) und ein Lumen (220) in Fluidverbindung mit der ersten und zweiten Öffnung der zwei oder mehr Öffnungen aufweist,
wobei die Ventilanordnung als Reaktion auf eine Verwendung der Ventilanordnung zur Steuerung eines Fluidstroms durch einen Ventilschacht (106, 206, 306) zum Übergehen von einem ersten Zustand (210-1, 410-1, 510-1, 610-1) in einen zweiten Zustand (210-2, 410-2, 510-2, 610-2) konfiguriert ist, und wobei der zweite Zustand der Ventilanordnung eine erneute Verwendung der Ventilanordnung zum Steuern eines Fluidstroms durch Ventilschächte verhindert,
wobei die Fluidverbindung zwischen der ersten und zweiten Öffnung über das Lumen im zweiten Zustand blockiert ist.

2. Ventilanordnung nach Anspruch 1, wobei das Lumen (220) eine Übergangsablagerung (222, 522) aufweist, die ein Material aufweist, das dazu konfiguriert ist, sich auszudehnen und einen Teil des Lumens zu füllen, wenn das Material einer Flüssigkeit ausgesetzt wird.

3. Ventilanordnung nach einem der Ansprüche 1 oder 2, wobei der proximale und der distale Abschnitt (328-1, 428-1, 528-1, 328-2, 428-2, 528-2) des Ventilschafts voneinander abgekoppelt werden, um von dem ersten Zustand (410-1, 510-1) in den zweiten Zustand (410-2, 510-2) überzugehen.

4. Ventilanordnung nach einem der Ansprüche 1 bis 3, wobei der proximale und der distale Abschnitt (328-1, 428-1, 328-2, 428-2) des Ventilschafts im ersten Zustand (410-1) durch ein Haltegestänge (330-1, 330-2, 430) und ein Trenngestänge (332-1, 332-2, 432-1, 432-2) verbunden sind und der proximale und der distale Abschnitt des Ventilschafts im zweiten Zustand (410-2) durch das Haltegestänge und nicht durch das Trenngestänge verbunden sind.

5. Ventilanordnung nach Anspruch 4, wobei das Trenngestänge (332-1, 332-2, 432-1, 432-2) oder das Haltegestänge (330-1, 330-2, 430) innerhalb des Lumens (220) angeordnet ist.

6. Ventilanordnung nach einem der Ansprüche 4 bis 5, wobei das Trenngestänge oder das Haltegestänge außerhalb des Lumens (220) angeordnet ist.

7. Ventilanordnung nach einem der Ansprüche 4 bis 6, wobei das Trenngestänge (332-1, 332-2, 432-1, 432-2) eine Schlaufe aufweist, die an einem distalen Ende des proximalen Abschnitts (328-1, 428-1) des Ventilschafts oder an einem proximalen Ende des distalen Abschnitts (328-2, 428-2) des Ventilschafts angeordnet ist.

8. Ventilanordnung nach einem der Ansprüche 1 bis 7, wobei eine Verwendung der Ventilanordnung zum Steuern eines Fluidstroms durch einen Ventilschacht (106, 206, 306) eines oder mehrere der folgenden Elemente aufweist: Einsetzen des Ventilschafts (104, 204, 304, 404, 504, 604) in den Ventilschacht, Steuern eines Fluidstroms durch den Ventilschacht und Entfernen des Ventilschafts aus dem Ventilschacht.

9. Ventilanordnung nach einem der Ansprüche 1 bis 8, wobei der proximale Abschnitt (328-1) des Ventilschafts durch eine geformte Schwachstelle im ersten Zustand relativ zum distalen Abschnitt (328-2) des Ventilschafts fixiert ist und der proximale Abschnitt des Ventilschafts im zweiten Zustand verschiebbar mit dem distalen Abschnitt des Ventilschafts gekoppelt ist.

10. Ventilanordnung nach Anspruch 9, wobei eine Verwendung der Ventilanordnung zum Steuern eines Fluidstroms durch den Ventilschacht (306) die geformte Schwachstelle bricht, um die Ventilanordnung von dem ersten Zustand in den zweiten Zustand zu überführen.

11. Ventilanordnung nach einem der Ansprüche 1 bis 10, wobei der proximale Abschnitt (528-1) des Ventilschafts relativ zum distalen Abschnitt (528-2) des Ventilschafts im ersten Zustand (510-1) durch eine Übergangsablagerung (522) fixiert ist und der proximale Abschnitt des Ventilschafts im zweiten Zustand (510-2) verschiebbar mit dem distalen Abschnitt des Ventilschafts gekoppelt ist.

12. Ventilanordnung nach Anspruch 11, bei der ein Einwirken der Übergangsablagerung (522) auf eine Flüssigkeit durch eine Verwendung der Ventilanordnung zum Steuern eines Fluidstroms durch den Ventilschacht, die Übergangsablagerung auflöst, um die Ventilanordnung von dem ersten Zustand (510-1) in den zweiten Zustand (510-2) zu überführen.

13. Ventilanordnung nach einem der Ansprüche 1 bis 12, die mehrere radiale Schenkel (640) mit ersten und zweiten Enden aufweist, wobei die ersten Enden schwenkbar mit dem Ventilschaft (604) verbunden sind.

14. Ventilanordnung nach Anspruch 13, wobei die zweiten Enden in der Nähe des Ventilschafts (604) durch eine Haube (642) gehalten werden, die im ersten Zustand (610-1) verschiebbar mit dem Ventilschaft gekoppelt ist, und die zweiten Enden im zweiten Zustand (610-2) durch die Haube freigegeben werden.

## Revendications

1. Ensemble de valve (100, 200, 300A, 300B) pour contrôler l'écoulement de fluide à travers des puits de valve, l'ensemble de valve comprenant :
un élément d'interface (102, 202, 302) ; et
une tige de valve (104, 204, 304, 404, 504, 604) à laquelle l'élément d'interface peut être couplé de manière amovible, la tige de valve comprenant une partie proximale (328-1, 428-1, 528-1), une partie distale (328-2, 428-2, 528-2), deux orifices ou plus (112, 212) et une lumière (220) en communication de fluide avec des premier et deuxième orifices des deux orifices ou plus,
dans lequel l'ensemble de valve est configuré pour effectuer une transition d'un premier état (210-1, 410-1, 510-1, 610-1) à un deuxième état (210-2, 410-2, 510-2, 610-2) en réponse à l'utilisation de l'ensemble de valve pour contrôler l'écoulement de fluide à travers un puits de valve (106, 206, 306), et dans lequel le deuxième état de l'ensemble de valve empêche la réutilisation de l'ensemble de valve pour contrôler l'écoulement de fluide à travers les puits de valve,
dans lequel la communication de fluide entre les premier et deuxième orifices via la lumière est bloquée dans le deuxième état.

2. Ensemble de valve selon la revendication 1, dans lequel la lumière (220) comprend un dépôt de transition (222, 522) comprenant un matériau configuré pour expanser et remplir une partie de la lumière, lorsque le matériau est exposé à un liquide.

3. Ensemble de valve selon l'une quelconque des revendications 1 ou 2, dans lequel les parties proximale et distale (328-1, 428-1, 528-1, 328-2, 428-2, 528-2) de la tige de valve se découplent pour effectuer une transition du premier état (410-1, 510-1) au deuxième état (410-2, 510-2).

4. Ensemble de valve selon l'une quelconque des revendications 1 à 3, dans lequel les parties proximale et distale (328-1, 428-1, 328-2, 428-2) de la tige de valve sont raccordées à une liaison de retenue (330-1, 330-2, 430) et une liaison de séparation (332-1, 332-2, 432-1, 432-2) dans le premier état (410-1) et les parties proximale et distale de la tige de valve sont raccordées par la liaison de retenue et pas par la liaison de séparation dans le deuxième état (410-2).

5. Ensemble de valve selon la revendication 4, dans lequel la liaison de séparation (332-1, 332-2, 432-1, 432-2) ou la liaison de retenue (330-1, 330-2, 430) est disposée à l'intérieur de la lumière (220).

6. Ensemble de valve selon l'une quelconque des revendications 4 à 5, dans lequel la liaison de séparation ou la liaison de retenue est disposée à l'extérieur de la lumière (220).

7. Ensemble de valve selon l'une quelconque des revendications 4 à 6, dans lequel la liaison de séparation (332-1, 332-2, 432-1, 432-2) comprend une boucle disposée sur une extrémité distale de la partie proximale (328-1, 428-1) de la tige de valve ou sur une extrémité proximale de la partie distale (328-2, 428-2) de la tige de valve.

8. Ensemble de valve selon l'une quelconque des revendications 1 à 7, dans lequel l'utilisation de l'ensemble de valve pour contrôler l'écoulement de fluide à travers un puits de valve (106, 206, 306) comprend un ou plusieurs parmi l'insertion de la tige de valve (104, 204, 304, 404, 504, 604) dans le puits de valve, le contrôle d'écoulement de fluide à travers le puits de valve et le retrait de la tige de valve du puits de valve.

9. Ensemble de valve selon l'une quelconque des revendications 1 à 8, dans lequel la partie proximale (328-1) de la tige de valve est fixe par rapport à la partie distale (328-2) de la tige de valve par un point de faiblesse moulé dans le premier état, et la partie proximale de la tige de valve est couplée, de manière coulissante, à la partie distale de la tige de valve dans le deuxième état.

10. Ensemble de valve selon la revendication 9, dans lequel l'utilisation de l'ensemble de valve pour contrôler l'écoulement de fluide à travers le puits de valve (306) casse le point de faiblesse moulé pour faire effectuer une transition à l'ensemble de valve du premier état au deuxième état.

11. Ensemble de valve selon l'une quelconque des revendications 1 à 10, dans lequel la partie proximale (528-1) de la tige de valve est fixe par rapport à la partie distale (528-2) de la tige de valve par un dépôt de transition (522) dans le premier état (510-1), et la partie proximale de la tige de valve est couplée, de manière coulissante, à la partie distale de la tige de valve dans le deuxième état (510-2).

12. Ensemble de valve selon la revendication 11, dans lequel l'exposition du dépôt de transition (522) à un liquide à partir de l'utilisation de l'ensemble de valve pour contrôler l'écoulement de fluide à travers le puits de valve dissout le dépôt de transition pour que l'ensemble de valve effectue une transition du premier état (510-1) au deuxième état (510-2).

13. Ensemble de valve selon l'une quelconque des revendications 1 à 12, comprenant une pluralité de pattes radiales (640) avec des premières et deuxièmes extrémités, dans lequel les premières extrémités sont raccordées, de manière pivotante, à la tige de valve (604).

14. Ensemble de valve selon la revendication 13, dans lequel les deuxièmes extrémités sont retenues à proximité de la tige de valve (604) par une coiffe (642) couplée, de manière coulissante, à la tige de valve dans le premier état (610-1) et les deuxièmes extrémités sont libérées par la coiffe dans le deuxième état (610-2).
